# EUROPÄISCHE PATENTSCHRIFT

(11) **EP 3 160 541 B1**
(45) Veröffentlichungstag und Bekanntmachung des Hinweises auf die Patenterteilung: **05.12.2018**
(21) Anmeldenummer: 15729811.8
(22) Anmeldetag: 15.06.2015
(51) Int. Cl.: A61M 5/14, A61M 5/168

(54) **VORRICHTUNG ZUM VERABREICHEN VON FLUID AN EINEN PATIENTEN**
DEVICE FOR ADMINISTERING FLUID TO A PATIENT
DISPOSITIF PERMETTANT D'ADMINISTRER UN FLUIDE À UN PATIENT

(30) Priorität: 25.06.2014 DE 102014212237
(43) Veröffentlichungstag der Anmeldung: 03.05.2017
(73) Patentinhaber: B. Braun Melsungen AG, 34212 Melsungen (DE)
(72) Erfinder: HASLBECK, Karsten, 34212 Melsungen (DE)
(74) Vertreter: Patentanwälte Ruff, Wilhelm, Beier, Dauster & Partner mbB
(86) Internationale Anmeldenummer: PCT/EP2015/063289
(87) Internationale Veröffentlichungsnummer: WO 2015/197397

(56) Entgegenhaltungen:
- US-A1- 2012 053 556
- US-A1- 2013 310 770
- US-B2- 6 981 967

## Beschreibung

Die Erfindung betrifft eine Vorrichtung zum Verabreichen von Fluid an einen Patienten.

Derartige Vorrichtungen sind insbesondere in der patientenkontrollierten Analgese (PCA - patient controlled analgesia) von Bedeutung. Die Analgese hat zum Ziel, Patienten kontinuierlich mit einem Schmerzmittel zu versorgen, um Schmerzen zu unterdrücken. Zur Linderung von Schmerzattacken, die über ein durchschnittliches Schmerzlevel hinaus gehen, ist es bekannt, dem Patienten ein Betätigungselement zur Verfügung zu stellen, dessen Betätigung die Verabreichung einer zusätzlichen Dosis von Schmerzmittel (Bolus) auslöst.

Hierzu ist ein Bolusreservoir vorgesehen, in dem die Fluiddosis für den Bolus enthalten ist. Das Bolusreservoir kann in einem Bolusflussweg angeordnet sein, der die Fluidquelle mit einem Patientenanschluss verbindet. Ein kontinuierlicher Durchflussweg verbindet die Fluidquelle mit dem Patientenanschluss, um das Fluid der Fluidquelle kontinuierlich dem Patienten zuzuführen. Sobald die Verabreichung eines Bolus ausgelöst wird, wird dieser aus dem Bolusreservoir über den Bolusflussweg ebenfalls dem Patientenanschluss zugeführt.

EP 1 395 315 B1, US2013/0310770 und US 6,981,967 B2 beschreiben eine Vorrichtung zur Verabreichung von großvolumigen Boli nach dem Oberbegriff von Anspruch 1. Der manuell einstellbare Durchflussregler ist in dem kontinuierlichen Durchflussweg enthalten und so konfiguriert, dass er eine Flussrate in dem kontinuierlichen Durchflussweg regelt. Bei dieser Lösung besteht ein Nachteil darin, dass mögliche Druckspitzen des geförderten Fluids das Bolusreservoir und/oder den Bolusflussweg beschädigen können.

Der Erfindung liegt die Aufgabe zugrunde, eine verbesserte Fluidverabreichungsvorrichtung zu schaffen, bei der das Bolusreservoir gegen Beschädigung durch Überdruck geschützt ist.

Die erfindungsgemäße Vorrichtung ist definiert durch die Merkmale von Anspruch 1.

Demnach ist der manuell einstellbare Durchflussregler in dem einstellbaren Bolusflussweg und nicht in dem kontinuierlichen Durchflussweg angeordnet. Der Flussregler regelt also nicht die Flussrate in dem kontinuierlichen Durchflussweg, sondern die Flussrate in dem Bolusflussweg. Der Durchflussregler ist in dem Bolusflussweg parallel zu dem Bolusreservoir angeordnet. Der Bolusflussweg weist dabei zwei parallel zueinander verlaufende Teilpfade auf, von denen der eine das Bolusreservoir und der andere den manuell einstellbaren Durchflussregler aufweist. Das heißt, dass der manuell einstellbare Durchflussregler die Fluidquelle und das Patientenende über den Bolusflussweg miteinander verbindet.

Im Fall einer Druckspitze, die beispielsweise bei einem Zusammendrücken der Fluidquelle (Pumpenreservoir) auftreten kann, wird die Flussrate durch den kontinuierlichen Durchflussweg erhöht. Die Flussrate durch den kontinuierlichen Durchflussweg wird dabei nicht von dem manuell einstellbaren Durchflussregler begrenzt, so dass die Druckspitze über den kontinuierlichen Durchflussweg entweichen kann, ohne das Bolusreservoir zu beschädigen. Der manuell einstellbare Durchflussregler ist in dem Bolusflussweg und nicht in dem kontinuierlichen Durchflussweg angeordnet.

Vorteilhafterweise ist die Flussrate durch den kontinuierlichen Durchflussweg fest vorgegeben, zum Beispiel durch einen Flussbegrenzer, und nicht manuell einstellbar. Insbesondere in dem kontinuierlichen Durchflussweg ist kein manuell einstellbarer Durchflussregler vorgesehen und der Durchfluss nicht veränderbar. Die Flussrate durch den kontinuierlichen Durchflussweg kann im Bereich zwischen 0,1 ml/h (Milliliter pro Stunde) und 2 ml/h liegen.

Der manuell einstellbare Durchflussregler kann den Durchfluss durch den Bolusflussweg in einem Bereich zwischen 0 ml/h und 14 ml/h einstellen.

Der Bolusflussweg kann zwei Teilpfade aufweisen, von denen der eine Teilpfad dem Bolusreservoir und der andere Teilpfad dem manuell einstellbaren Durchflussregler zugeordnet ist. Der Unterschied zwischen dem Teilpfad des Bolusflussweges mit dem manuell einstellbaren Durchflussregler und dem kontinuierlichen Durchflussweg ist darin zu sehen, dass die Flussrate durch den kontinuierlichen Durchflussweg in der Regel kleiner ist als durch den Teilpfad mit dem manuell einstellbaren Durchflussregler.

Der kontinuierliche Durchflussweg kann mit einer Flussdrossel versehen sein. Bei der Flussdrossel kann es sich beispielsweise um den Flusswiderstand des kontinuierlichen Durchflussweges oder um ein separates Bauteil handeln.

Bei der Fluidquelle handelt es sich vorzugsweise um eine Fluidförderpumpe, die das dem Patienten zu verabreichende Fluid mit einem Druck beaufschlagt, der größer ist als atmosphärischer Druck.

Bei dem Patientenende kann es sich um einen Anschluss handeln, der mit einem Katheter oder einer Injektionsnadel verbindbar ist.

Der kontinuierliche Durchflussweg und der Bolusflussweg sind vorteilhafterweise über einen gemeinsamen Eingangspfad mit der Fluidquelle verbunden. Der kontinuierliche Durchflussweg und der Bolusflussweg sind vorteilhafterweise über einen Ausgangspfad mit dem Patientenende verbunden. Der kontinuierliche Durchflussweg und der Bolusflussweg zweigen aus dem Eingangspfad ab und/oder münden in den Ausgangspfad.

In dem Eingangspfad und/oder in dem Ausgangspfad kann ein Flussindikator vorgesehen sein, der dazu ausgebildet ist, anzuzeigen, ob durch den betreffenden Pfad Fluid strömt. Bei dem Flussindikator kann es sich im einfachsten Fall um ein durchsichtiges Röhrchen handeln, durch welches Fluid in dem betreffenden Pfad erkennbar ist. Alternativ kann es sich auch um eine Anzeige handeln, die die Flussrate anzeigt. Denkbar ist als Flussindikator auch ein Prisma, dessen Reflexions-/Brechungseigenschaften sich ändern, wenn das Prisma von einer Flüssigkeit umströmt wird.

Der kontinuierliche Durchflussweg enthält ein Überdruckventil welches einen Überdruck im System in ein Sammelreservoir abführen kann.

Manuell einstellbar ist nur die Flussrate des Bolusflussweges und nicht die Flussrate des kontinuierlichen Durchflussweges.

Das Bolusreservoir ist mit einem Betätigungselement versehen, welches einem Bediener gestattet, durch Betätigen des Betätigungselements eine Bolusgabe auszulösen. Beispielsweise kann es sich bei dem Betätigungselement um einen Knopf handeln, der einen federbetätigten Kolben freigibt, wobei durch Verschieben des Kolbens Fluid aus dem Bolusreservoir herausgepresst wird. Alternativ kann es sich bei dem Bolusreservoir um ein elastisch expandierendes Fluidreservoir zum Beispiel als Ballon handeln, der sich bei einströmendem Fluid ausdehnt. Die Sperre wird dabei automatisch gelöst und das Betätigungselement zur Betätigung freigegeben, sobald eine vorgegebene Fluidmenge in das Bolusreservoir eingeströmt ist. Eine solche Sperre ist in der deutschen Patentanmeldung 10 2013 213 529.7 beschrieben, deren Inhalt durch Bezugnahme in die vorliegende Beschreibung übernommen wird.

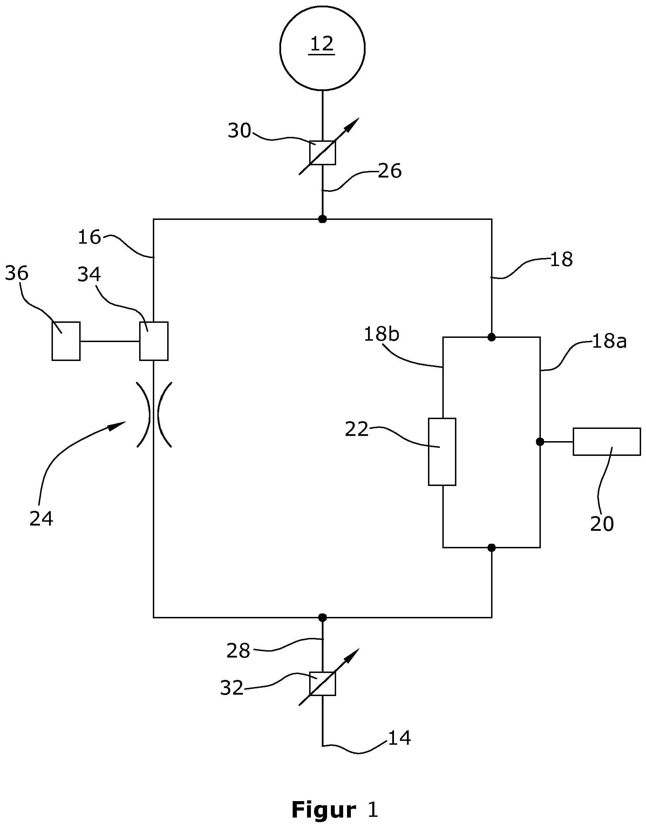

Im Folgenden wird anhand der Figur ein Ausführungsbeispiel näher erläutert. Die Figur zeigt eine schematische Darstellung des Ausführungsbeispiels.

Die Fluidquelle 12 ist eine Fluidförderpumpe und über einen Eingangspfad fluidleitend mit dem kontinuierlichen Durchflussweg 16 und dem Bolusflussweg 18 verbunden. Der kontinuierliche Durchflussweg 16 und der Bolusflussweg 18 zweigen als separate Flusswege von dem Eingangspfad 26 ab. Der kontinuierliche Durchflussweg 16 und der Bolusflussweg 18 sind in Bezug auf die Fluidquelle 12 und das Patientenende 14 parallel zueinander angeordnet.

Der kontinuierliche Durchflussweg 16 ist mit einer Flussdrossel 24 versehen, die die Flussrate des kontinuierlichen Durchflussweges 16 fest vorgibt. Die Flussrate durch den kontinuierlichen Durchflussweg 16 ist nicht veränderbar. Der kontinuierliche Durchflussweg enthält ein Überdruckventil 34 welches einen Überdruck im System in ein Sammelreservoir 36 abführt.

Der Bolusflussweg 18 weist zwei parallel zueinander angeordnete Teilpfade 18a, 18b auf. Von dem ersten Teilpfad 18a zweigt das Bolusreservoir 20 ab. Das Bolusreservoir 20 ist ein Fluidreservoir zur Aufnahme einer Bolusdosis von Fluid, die dem Patienten impulsartig verabreicht werden kann. Bei dem Fluid handelt es sich typischerweise um ein Schmerzmittel. Bei der patientenkontrollierten Analgese (PCA) ist es bekannt, dass ein Patient die Verabreichung eines Bolus durch Betätigen eines entsprechenden Betätigungsorgans (Schalter) selbst auslösen kann. Dabei ist es ebenfalls bekannt, dass der Zeitabstand zum Verabreichen eines erneuten Bolus, nachdem ein Bolus bereits ausgelöst worden ist, festgelegt ist. Dadurch wird eine Überdosierung vermieden. Hierzu kann in dem Teilpfad 18a ein Flussbegrenzer vorgesehen sein, der die Dauer zur Befüllung des Bolusreservoirs 20 mit Fluid vorgibt. Das Bolusreservoir 20 ist dazu ausgebildet, einen Bolus erst dann auslösen zu können, wenn das Reservoir 20 bis zu einem bestimmten Grad gefüllt ist.

In dem zweiten Teilpfad 18b des Bolusflussweges 18 ist ein manuell einstellbarer Durchflussregler 22 vorgesehen. In dem kontinuierlichen Durchflussweg 16 ist hingegen kein manuell einstellbarer Durchflussregler vorgesehen. Der manuell einstellbare Durchflussregler 22 ist dazu ausgebildet, eine Flussrate im Bereich zwischen 0 ml/h und 14 ml/h einzustellen.

Die beiden Teilpfade 18a, 18b münden in eine gemeinsame Leitung des Bolusflussweges 18. Der kontinuierliche Durchflussweg 16 und der Bolusflussweg 18 münden in einen gemeinsamen Ausgangspfad 28, der mit dem Patientenende 14 fluidleitend verbunden ist. In dem Ausgangspfad 28 ist ein Fluidindikator 32 vorgesehen. Der Fluidindikator 32 gibt visuell an, ob Fluid durch den Ausgangspfad 28 strömt.

## Patentansprüche

1. Vorrichtung zur Verabreichung von Fluid an einen Patienten, mit:
einer Fluidquelle (12) zum Bereitstellen von Fluid,
einem Patientenende (14), welches zum Anschließen eines Patienten ausgebildet ist,
einem die Fluidquelle (12) und das Patientenende (14) verbindenden kontinuierlichen Durchflussweg (16) zum kontinuierlichen Fördern von Fluid von der Fluidquelle (12) zum Patientenende (14),
einem mit der Fluidquelle (12) und dem Patientenende (14) verbundenen und parallel zu dem kontinuierlichen Durchflussweg (16) angeordneten Bolusflussweg (18) zum Fördern einer Bolusdosis von Fluid, wobei der Bolusflussweg (18) mit einem Bolusreservoir (20) zum Speichern der Bolusdosis versehen ist und
einem manuell von einer Bedienperson einstellbaren Durchflussregler (22), der zum manuellen Einstellen einer Flussrate ausgebildet ist,
**dadurch gekennzeichnet,**
**dass** der manuell einstellbare Durchflussregler (22) in dem Bolusflussweg (18) parallel zu dem Bolusreservoir (20) angeordnet ist, und
**dass** der kontinuierliche Durchflussweg (16) ein Überdruckventil (34) enthält.

2. Vorrichtung nach einem der vorhergehenden Ansprüche, **dadurch gekennzeichnet, dass** der Bolusflussweg (18) zwei parallele Teilpfade (18a, 18b) aufweist, wobei das Bolusreservoir nur mit dem einen Teilpfad (18a) und der manuell einstellbare Durchflussregler (22) nur mit dem anderen Teilpfad (18b) verbunden ist.

3. Vorrichtung nach einem der vorhergehenden Ansprüche, **dadurch gekennzeichnet, dass** die Fluidquelle (12) eine Fluidförderpumpe aufweist.

4. Vorrichtung nach einem der vorhergehenden Ansprüche, **dadurch gekennzeichnet, dass** der kontinuierliche Durchflussweg einen Flussbegrenzer (24) aufweist.

5. Vorrichtung nach einem der vorhergehenden Ansprüche, **dadurch gekennzeichnet, dass** der kontinuierliche Durchflussweg (16) und der Bolusflussweg (18) über einen gemeinsamen Eingangspfad (26) mit der Fluidquelle (12) und/oder über einen gemeinsamen Ausgangspfad (28) mit dem Patientenende (14) verbunden sind.

6. Vorrichtung nach dem vorhergehenden Anspruch, **dadurch gekennzeichnet, dass** der Eingangspfad (26) und/oder der Ausgangspfad (28) einen Flussindikator (30, 32) zum Anzeigen des Durchflusses durch den Eingangspfad bzw. den Ausgangspfad aufweisen.

7. Vorrichtung nach einem der vorhergehenden Ansprüche, **dadurch gekennzeichnet, dass** die Flussrate durch den kontinuierlichen Durchflussweg (16) fest vorgegeben und nicht manuell veränderbar ist.

8. Vorrichtung nach einem der vorhergehenden Ansprüche, **dadurch gekennzeichnet, dass** die Flussrate durch den kontinuierlichen Durchflussweg (16) im Bereich zwischen 0,1 ml/h und 2 ml/h beträgt.

9. Vorrichtung nach einem der vorhergehenden Ansprüche, **dadurch gekennzeichnet, dass** der manuell einstellbare Durchflussregler das Einstellen einer Flussrate im Bereich zwischen 0 ml/h und 14 ml/h ermöglicht.

10. Vorrichtung nach einem der vorhergehenden Ansprüche, **dadurch gekennzeichnet, dass** der Bolusflussweg (18) einen Flussbegrenzer zum Vorgeben der Dauer zum Befüllen des Bolusreservoirs (20) aufweist.

11. Vorrichtung nach einem der vohergehenden Ansprüche, **dadurch gekennzeichnet, dass** das Bolusreservoir (20) ein Betätigungselement zum Auslösen eines Bolus und eine die Betätigung des Betätigungselements verhindernde Sperre aufweist, die nach Einströmen einer vorgegebenen Fluidmenge in das Bolusreservoir gelöst wird.

## Claims

1. Device for administering fluid to a patient, comprising:
a fluid source (12) for providing fluid,
a patient end (14) which is adapted to be connected to a patient,
a continuous throughflow path (16) connecting the fluid source (12) and the patient end (14) for continuously supplying fluid from the fluid source (12) to the patient end (14),
a bolus flow path (18) connected to the fluid source (12) and the patient end (14) and arranged in parallel to the continuous throughflow path (16) for supplying a bolus dose of fluid, wherein the bolus flow path (18) is provided with a bolus reservoir (20) for storing the bolus dose and
a manually adjustable throughflow controller (22), adjustable by an operating person, which is designed for manual adjustment of a flow rate,
**characterized in that**
the manually adjustable throughflow controller (22) is arranged in the bolus flow path (18) parallel to the bolus reservoir (20), and **in that**
the continuous throughflow path (16) includes a pressure relief valve (34).

2. Device according to any of the preceding claims, **characterized in that** the bolus flow path (18) has two parallel partial paths (18a, 18b), the bolus reservoir being connected only to the one partial path (18a) and the manually adjustable throughflow controller (22) being connected only to the other partial path (18b).

3. Device according to any of the preceding claims, **characterized in that** the fluid source (12) comprises a fluid delivery pump.

4. Device according to any of the preceding claims, **characterized in that** the continuous throughflow path comprises a flow limiter (24).

5. Device according to any of the preceding claims, **characterized in that** the continuous throughflow path (16) and the bolus flow path (18) are connected with the fluid source (12) via a common inlet path (26) and/or with the patient end (14) via a common outlet path (28).

6. Device according to any of the preceding claims, **characterized in that** the inlet path (26) and/or the outlet path (28) comprise a flow indicator (30, 32) to indicate a flow through the inlet path or the outlet path, respectively.

7. Device according to any of the preceding claims, **characterized in that** the flow rate through the continuous throughflow path (16) is predefined and cannot be varied manually.

8. Device according to any of the preceding claims, **characterized in that** the flow rate through the continuous throughflow path (16) is in a range between 0.1 ml/h and 2 ml/h.

9. Device according to any of the preceding claims, **characterized in that** the manually adjustable throughflow controller allows the adjustment of a flow rate in a range between 0 ml/h and 14 ml/h.

10. Device according to any of the preceding claims, **characterized in that** the bolus flow path (18) comprises a flow limiter to predefine the time for filling the bolus reservoir (20).

11. Device according to any of the preceding claims, **characterized in that** the bolus reservoir (20) has an actuation element for triggering a bolus and a lock preventing the actuation of the actuation element, said lock being released after a predefined amount of fluid has flown into the bolus reservoir.

## Revendications

1. Appareil destiné à l'administration de fluide à un patient, doté :
d'une source de fluide (12) destinée à préparer le fluide,
d'une extrémité patient (14), conçue pour se raccorder à un patient;
d'une voie d'écoulement en continu (16) connectant la source de fluide (12) et l'extrémité patient (14) pour le convoyage continu de fluide de la source de fluide (12) à l'extrémité patient (14),
d'une voie d'écoulement de bolus (18) connectée à la source de fluide (12) et à l'extrémité patient (14) et parallèle à la voie d'écoulement en continu (16) pour le convoyage d'un bolus de fluide, dans lequel la voie d'écoulement de bolus (18) est prévue dotée d'un réservoir de bolus (20) pour stocker la dose de bolus et
d'un régulateur de débit (22) réglable manuellement par un opérateur, conçu pour le réglage manuel du débit,
**caractérisé en ce que** le régulateur de débit (22) réglable manuellement est agencé dans la voie d'écoulement de bolus (18) parallèlement au réservoir de bolus (20), et
**en ce que** la voie d'écoulement en continu (16) comprend une valve de surpression (34).

2. Appareil selon l'une des revendications ci-dessus, **caractérisé en ce que** la voie d'écoulement de bolus (18) comporte deux chemins partiels parallèles (18a, 18b), dans lequel le réservoir de bolus est connecté uniquement avec l'un des chemins partiels (18a) et le régulateur de débit (22) réglable manuellement est connecté uniquement avec l'autre chemin partiel (18b).

3. Appareil selon l'une des revendications ci-dessus, **caractérisé en ce que** la source de fluide (12) comporte une pompe de transfert de fluide.

4. Appareil selon l'une des revendications ci-dessus, **caractérisé en ce que** la voie d'écoulement en continu comporte un limiteur de débit (24).

5. Appareil selon l'une des revendications ci-dessus, **caractérisé en ce que** la voie d'écoulement en continu (16) et la voie d'écoulement de bolus (18) sont connectées à la source de fluide (12) au moyen d'un chemin d'entrée commun (26) et/ou sont connectées à l'extrémité patient (14) au moyen d'un chemin de sortie commun (28).

6. Appareil selon la revendication ci-dessus, **caractérisé en ce que** le chemin d'entrée commun (26) et/ou le chemin de sortie commun (28) comportent un indicateur de débit (30, 32) destiné à afficher le débit au travers du chemin d'entrée commun et/ou du chemin de sortie commun.

7. Appareil selon l'une des revendications ci-dessus, **caractérisé en ce que** le débit au travers de la voie d'écoulement en continu (16) est prédéterminé et ne peut être modifié manuellement.

8. Appareil selon l'une des revendications ci-dessus, **caractérisé en ce que** le débit au travers de la voie d'écoulement en continu (16) est situé dans un intervalle compris entre 0,1 ml/h et 2 ml/h.

9. Appareil selon l'une des revendications ci-dessus, **caractérisé en ce que** le régulateur de débit réglable manuellement permet de régler le débit dans un intervalle compris entre 0 ml/h et 14 ml/h.

10. Appareil selon l'une des revendications ci-dessus, **caractérisé en ce que** la voie d'écoulement de bolus (18) comporte un limiteur de débit destiné à prédéfinir la durée de remplissage du réservoir de bolus (20).

11. Appareil selon l'une des revendications ci-dessus, **caractérisé en ce que** le réservoir de bolus (20) comporte un élément d'actionnement destiné à déclencher un bolus et un verrou empêchant l'actionnement de l'élément d'actionnement qui est libéré après afflux d'une quantité prédéfinie de fluide dans le réservoir de bolus.
